# EUROPEAN PATENT APPLICATION

(11) **EP 4 588 890 A1**
(43) Date of publication of application: **23.07.2025**
(21) Application number: 24382040.4
(22) Date of filing: 16.01.2024
(51) Int. Cl.: C01B 17/45, C07C 209/68, C07C 381/00, C07D 213/02, C07D 233/58

(54) **PROCESS FOR THE PREPARATION OF PENTAFLUOROSULFONOLATE SALTS COMPOSITION, COMPOSITION OBTAINED BY THE PROCESS AND THE USE OF THE COMPOSITION**

(71) Applicant: Universitat Rovira I Virgili, 43003 Tarragona (ES)
(72) Inventor: MESTRE VENTURA, Jordi, 43003 TARRAGONA (ES); BOUTUREIRA MARTÍN, Omar, 43003 TARRAGONA (ES); BERNÚS PÉREZ, Miguel, 43003 TARRAGONA (ES)
(74) Representative: Herrero & Asociados, S.L.

(57) **Abstract**

The present invention relates to a process for the preparation of pentafluorosulfonolate salts composition, the composition obtained in the process and its use. The composition comprising the pentafluorosulfonolate salts are useful for incorporating the pentafluorosulfanolate fragment into organic molecules using SFsO anionic species as reagents.

## Description

### FIELD OF THE INVENTION

The present invention relates to a process for the preparation of pentafluorosulfonolate salts composition, the composition obtained in the process and its use. The composition comprising the pentafluorosulfonolate salts are useful for incorporating the pentafluorosulfanolate fragment into organic molecules using SFsO anionic species as reagents.

### BACKGROUND OF THE INVENTION

In the context of SF₅ chemistry, research has focused on the construction on C-SF₅ bonds. However, the combination of SF₅ with other atoms remains vastly unexplored.

In this context, the pentafluoro-λ⁶-sulfanolate fragment (SF₅O⁻) emerges as a promising motif for medicinal, agrochemical chemistry, and material science. This substituent is structurally related to the SF₅ group where an octahedral geometry is adopted. Sulfur is surrounded by four fluorine atoms in a plane, and the two axial positions are occupied by an additional fluorine atom and the remaining oxygen.

This anion was first synthesized in 1960 by Smith and Englehardt and characterized as a CsF·SF₄O adduct. In fact, Lustig and Ruff later corrected the structure and recharacterized it as a the SF₅O⁻ anion, which has an octahedral arrangement similar to that of SF₅ fragments.

Currently, this is the only method for the preparation of pentafluorosulfanolate salts, which is based on the reaction of a fluoride source with gaseous thionyl tetrafluoride (SOF₄). This gaseous reagent is not commercially available, and its synthesis requires the use of toxic and very reactive gases in a specialized reactor. The European patent EP3027589 defines a process for the preparation of fluorinated compounds having at least one SF₅O⁻ group, said process comprising the step of reacting SOF₄ with a fluorinated 3- or 4-membered cyclic ether in the presence of a fluoride catalyst, wherein the catalyst is selected from: the group consisting of the alkali metal fluorides, the alkali-earth metal fluorides, the quaternary ammonium fluorides and silver fluoride. The poor stability of the SF₅O⁻ anion is inferred by its dynamic equilibrium with thionyl tetrafluoride (SOF₄) and fluoride anion.

In general, the chemistry of SF₅O⁻ remains largely unexplored due to synthetic challenges in obtaining suitable precursors. The need for specialized equipment and careful handling of reactive intermediates hampers the development of methodologies that could open opportunities to exploit this motif in different areas of the chemical sciences.

### SUMMARY OF THE INVENTION

The first objective of the present invention is to provide a simple and reproducible methodology to generate a composition comprising SF₅O⁻ salts.

The present disclosure is directed to a method for obtaining a composition comprising SF₅O⁻ salts, wherein the SF₅O⁻ anion remains stable within the composition. The described process yields a composition that can be effectively employed as a reagent in reactions aimed at incorporating the SF₅O⁻ compound into other compounds. The stability of the SF₅O⁻ anion in this composition implies its versatile application in various chemical reactions, providing a valuable tool for synthesizing and modifying compounds in different fields of chemistry.

The process defined in the present invention implies accessible and inexpensive starting materials. The process does not require the use of specialized equipment, and it can be performed in a common synthetic laboratory. The objective of the present disclosure is to provide a simple and reproducible process to generate a synthetically useful SF₅O⁻ salts.

Therefore, the first aspect of the disclosure refers to the process for the preparation of a composition comprising pentafluoro sulfanolate salts of general Formula (I)

Zⁿ⁺(SF₅O⁻)ₙ Formula (I)

n is selected from 1 or 2, the process comprising a step of reacting a first reactant of general Formula (II)

SF₅X Formula (II)

wherein X is a halogen, with a second reactant being an inorganic base wherein the cation is monovalent or divalent; and with a third reactant defines by the formula ZY or ZY₂, wherein Y is a halogen and Z represents a cation and is selected from: a cation of the formula R₁R₂R₃R₄N⁺ or R₁R₂R₃R₄P⁺, an imidazolium cation, a pyridinium cation, and/or bicycle (C₇-C₁₁)alkyl cation wherein 2 or 3 atoms of carbon atoms are substituted by 2 or 3 nitrogen atoms, wherein each of R₁, R₂, R₃, and R₄ independently is a C₁-C₆ alkyl, a C₇-C₁₂ arylalkyl, or a C₆-C₁₀ aryl, wherein the reaction is carried out in an aprotic solvent.

The stability of the SF₅O⁻ anion is improved incorporating the organic salt to ensure the presence of cations that make the pentafluorosulfonate salts sufficiently soluble in the reaction medium and avoid defluorination, preserving the stability of the SF₅O⁻ anion.

The term pentafluorosulfonate salts is also known as pentafluorooxosulfate, pentafluorosulfuranyloxy, or pentafluorosulfuroxide.

By the term "aprotic solvent" as used herein is meant a solvent which does not have an ionizable or donatable hydrogen. The solvent can be a polar aprotic solvent or an apolar aprotic solvent.

The composition obtained in the process described in the first aspect is suitable for direct application as it possesses favourable solubility of the SF₅O⁻ compound within the composition. Moreover, this process bypasses the use of unconventional SF₄O feedstock, commonly associated with SF₅O⁻. Additionally, the compounds generated during the composition process, others than SF₅O⁻ do not necessitate removal when employed as a reactant, thereby streamlining its utilization, and enhancing overall efficiency. Furthermore, since it is not necessary to isolate the compound, its stability is not compromised. Therefore, the second aspect of the invention refers to a composition obtainable by the process of the first aspect of the disclosure.

Finally, the third aspect of the disclosure refers to the use of the composition obtained in the process referred in the first aspect of the disclosure as a reactant in chemical reaction for the introduction of SF₅O⁻ into organic molecules.

### DETAILED DESCRIPTION OF THE INVENTION

As it is described above, the first aspect of the invention refers to the process for the preparation of a composition comprising pentafluoro sulfanolate salts of general Formula (I)

Zⁿ⁺(SF₅O⁻)ₙ Formula (I)

n is selected from 1 or 2, the process comprising a step of reacting a first reactant of general Formula (II)

SF₅X Formula (II)

wherein X is a halogen;
with a second reactant being an inorganic base wherein the cation is monovalent or divalent and;
with a third reactant defines by the formula ZY or ZY₂, wherein Y is a halogen and Z represents a cation and is selected from: alkali cation, alkaline earth cation, a transition cation or a cation of the formula R₁R₂R₃R₄N⁺ or R₁R₂R₃R₄P⁺, an imidazolium cation, a pyridinium cation, and/or bicycle(C₇-C₁₁)alkyl cation wherein 2 or 3 atoms of carbon atoms are substituted by 2 or 3 nitrogen atoms, wherein each of R₁, R₂, R₃, and R₄ independently is a C₁-C₆ alkyl, a C₇-C₁₂ arylalkyl, or a C₆-C₁₀ aryl, wherein the reaction is carried out in a aprotic solvent.

In a preferred embodiment of the process the halogen is selected from Cl, Br, I.

In a preferred embodiment the inorganic base having monovalent or divalent cations derived from alkali metals, alkaline earth or transition metal, more preferably selected from the group consisting of: Li⁺, Na⁺, K⁺, Rb⁺, Cs⁺, Fr⁺, Be²⁺, Mg²⁺, Ca²⁺, Sr²⁺, Ba²⁺, Zn²⁺, Cd²⁺. Particularly Cs⁺. Preferably, the counterion of inorganic selected from carbonate, phosphate, oxide, hydroxide, silicate anions, hydrogen carbonate, hydrogen phosphate, particularly carbonate.

Preferably the ratio of XSF₅ is 1:3 to 1:5 molar equivalents of the inorganic salts.

In a preferred embodiment the Z is selected from: Bu₄N⁺, Ph₄P⁺, 1-methyl-3-propyl-imidazolinium, 1-butylpyridinium, DABCO (1, 4-diazabicyclo[2.2.2]octane) mono or di protonated, TBD (1,5,7-triazabicyclo[4.4.0]dec-5-ene) mono or di protonated, DMAP (4-dimethylaminopyridine) mono or di protonated, DBU (1,8-Diazabicyclo[5.4.0]undec-7-ene) mono or di protonated. Particularly Bu₄N⁺Cl⁻. Preferably the ratio of XSF₅ is 1:1 to 1:7 molar equivalents of the organic salts and a more preferably is 1:2.5 to 1:5.

In a preferred embodiment the aprotic solvent is saturated with O₂. The O₂ dissolved in the reaction mixture could be instrumental in the formation of the product.

In the present disclosure the aprotic solvent could be selected between polar aprotic solvent and apolar aprotic solvent. More preferably the aprotic solvent is selected from: pentane, hexane, benzene, chloroform, diethyl ether, acetone, acetonitrile, dichloromethane, ethyl acetate, tetrahydrofuran. Particularly the solvent is selected from: hexane and acetonitrile. In a more preferred embodiment, the concentration of the solvent is in a range from 0.1M to 2M.

Particularly the temperature of the process is room temperature.

The term room temperature as used herein relates to a temperature between 20°C and 30°C, or between 25°C to 30°C.

Therefore, in a preferred embodiment the process is carried out at a temperature between 20°C and 30°C.

As it is said before the second aspect of the invention refers to a composition obtainable by the process of the first aspect of the disclosure and all the embodiment of the process.

Finally, the third aspect of the disclosure refers to the use of the composition obtained in the process referred in the first aspect of the disclosure as a reactant in chemical reaction for the introduction of SF₅O⁻ into organic molecules. Preferably the third aspect refers to the use of the composition in the process refereed in the first aspect in nucleophilic substitution reaction, wherein the nucleophilic compound is the SF₅O⁻ anion contain in the composition of the invention.

### Examples of the invention

### Example 1. Preparation of a composition comprising pentafluoro sulfanolate salts of general Formula (I) Zⁿ⁺(SF₅O⁻)ₙ

### General SF₅O⁻ salt preparation (GP-1)

In a 10 mL polypropylene (PP) tube containing a magnetic stir bar, cesium carbonate (326 mg, 1 mmol, 5 equiv) and a solution of dried quaternary salt are charged (0.2 mmol, 1 equiv). Then, additional anhydrous acetonitrile is added (1 mL). Next, a solution of SF₅Cl in hexane (0.12 M) is introduced (5 mL, 0.6 mmol, 3 equiv). The reaction is then submerged in a water bath and stirred for 6 h. To calculate the reaction conversion by ¹⁹F NMR, an aliquot of the crude is transferred to an NMR tube and 1,3-bis(trifluoromethyl)benzene is added as the internal standard.

### Tetrabutylammonium pentafluorosulfanolate

Following the general procedure GP-1, and using tetrabutylammonium chloride 2.44 M solution (82 µL, 0.2 mmol), SF₅Cl 0.11 M solution (5.5 mL, 3 equiv), Cs₂CO₃ (326 mg, 1 mmol, 5 equiv), and acetonitrile (1 mL), a solution of 2 was obtained in 65% yield. Yield was determined by ¹⁹F NMR using 1,3-bis(trifluoromethyl)benzene as the internal standard.

**¹⁹F NMR (CDC)₃, 376.5 MHz):** δ = 136.21 (p, *J*= 159.9 Hz, 1F), 92.90 (d, *J*= 159.9 Hz, 4F).

### 1-methyl-3-propyl-imidazolinium pentafluorosulfanolate

Following the general procedure GP-1 and using 1-methyl-3-propyl-imidazolinium chloride 0.87 M solution (230 µL, 0.2 mmol), SF₅Cl 0.12 M solution (4 mL, 2.5 equiv), Cs₂CO₃ (326 mg, 1 mmol, 5 equiv), and acetonitrile (1 mL), a solution of 1-methyl-3-propyl-imidazolinium pentafluorosulfanolate was obtained in 20% yield. Yield was determined by ¹⁹F NMR using 1,3-bis(trifluoromethyl)benzene as the internal standard. **¹⁹F NMR (CDC)₃, 376.5 MHz):** δ = 131.34 (p, J= 158.6 Hz, 1F), 90.44 (d, J= 158.6 Hz, 4F).

### 1-butylpyridinium pentafluorosulfanolate

Following the general procedure GP-1 and using 1-butylpyridinium bromide 0.37 M solution (540 µL, 0.2 mmol), SF₅Cl 0.12 M solution (4 mL, 2.5 equiv), Cs₂CO₃ (326 mg, 1 mmol, 5 equiv), and acetonitrile (1 mL), a solution of 1-butylpyridinium pentafluorosulfanolate was obtained in 17% yield. Yield was determined by ¹⁹F NMR using 1,3-bis(trifluoromethyl)benzene as the internal standard.

**¹⁹F NMR (CDCl₃, 376.5 MHz):** δ = 135.48 (p, J= 159.6 Hz, 1F), 92.56 (d, J= 159.6 Hz, 4F).

### Tetraphenylphosphonium pentafluorosulfanolate

Following the general procedure GP-1 and using tetraphenylphosphonium bromide 0.25 M solution (800 µL, 0.2 mmol), SF₅Cl 0.12 M solution (4 mL, 2.5 equiv), Cs₂CO₃ (326 mg, 1 mmol, 5 equiv), and acetonitrile (1 mL), a solution of tetraphenylphosphonium pentafluorosulfanolate was obtained in 17% yield. Yield was determined by ¹⁹F NMR using 1,3-bis(trifluoromethyl)benzene as the internal standard.

**¹⁹F NMR (CDC)₃, 376.5 MHz):** δ = 135.29 (p, *J*= 159.4 Hz, 1F), 92.47 (d, *J*= 159.4 Hz, 4F).

### Example 2. Reaction of SF₅O⁻ with 2-phenoxyethyl trifluoromethanesulfonate.

In an NMR tube containing a the composition obtained in Example 1 containing [Bu₄N][SF₅O] (0.05 mmol) in acetonitrile (0.5 mL), 2-phenoxyethyl trifluoromethanesulfonate (14 mg, 0.05 mmol, 1 equiv) was introduced using a Hamilton syringe. Next, the NMR tube was capped with a septum and heated at 45 °C for 18 h.

The product pentafluoro(2-phenoxyethoxy)-λ⁶-sulfane was obtained.

**¹⁹F NMR (CH₃CN, 376.5 MHz):** δ = 77.10 (p, *J*= 159.4 Hz, 1F), 60.66 (d, *J*= 159.4 Hz, 4F). **MS (GC-EI):** for (M)⁺ C₈H₉F₅O₂S⁺ (m/z): calc. 264.02; found 264.05.

## Claims

1. A process for the preparation of a composition comprising pentafluoro sulfanolate salts of general Formula (I)
Zⁿ⁺(SF₅O⁻)ₙ Formula (I)
n is selected from 1 or 2, the process is **characterized by** comprising a step of reacting a first reactant of general Formula (II)
SF₅X Formula (II)
wherein X is a halogen;
with a second reactant being an inorganic base wherein the cation is monovalent or divalent and;
with a third reactant defines by the formula ZY or ZY₂, wherein Y is a halogen and Z represents a cation and is selected from: alkali cation, alkaline earth cation, a transition cation or a cation of the formula R₁R₂R₃R₄N⁺ or R₁R₂R₃R₄P⁺, an imidazolium cation, a pyridinium cation, and/or bicycle(C₇-C₁₁)alkyl cation wherein 2 or 3 atoms of carbon atoms are substituted by 2 or 3 nitrogen atoms, wherein each of R₁, R₂, R₃, and R₄ independently is a C₁-C₆ alkyl, a C₇-C₁₂ arylalkyl, or a C₆-C₁₀ aryl, wherein the reaction is carried out in a aprotic solvent.

2. The process for the preparation of pentafluorosulfonolate salts according claim 1 **characterized by** the inorganic base having monovalent or divalent cations selected from: alkali metals, alkaline earth or transition metal, and wherein the counterion of inorganic base is selected from carbonate, phosphate, oxide, hydroxide, silicate anions, hydrogen carbonate, hydrogen phosphate, particularly carbonate.

3. The process for the preparation of pentafluorosulfonolate salts according claim 1 or claim 2 **characterized by** the ratio of XSF₅ is 1:3 to 1:5 molar equivalents of the inorganic salts.

4. The process for the preparation of pentafluorosulfonolate salts according any of the claims 1 to 3 **characterized by** Z is selected from: Bu₄N⁺, Ph₄P⁺, 1-methyl-3-propyl-imidazolinium, 1-butylpyridinium, DABCO (1, 4-diazabicyclo[2.2.2]octane) mono or di protonated, TBD (1,5,7-triazabicyclo[4.4.0]dec-5-ene) mono or di protonated, DMAP (4-dimethylaminopyridine) mono or di protonated, DBU (1,8-Diazabicyclo[5.4.0]undec-7-ene) mono or di protonated.

5. The process for the preparation of pentafluorosulfonolate salts according any of the claims 1 to 4 **characterized by** the ratio of XSF₅ is 1:1 to 1:7 molar equivalents of ZY or ZY₂.

6. The process for the preparation of pentafluorosulfonolate salts according any of the claims 1 to 5 **characterized by** the aprotic solvent is saturated with O₂.

7. The process for the preparation of pentafluorosulfonolate salts according any of the claims 1 to 5 **characterized by** the solvent is selected from: hexane and acetonitrile.

8. A composition obtainable by the process defined in any of the claims 1 to 7.

9. Use of the composition defined in claim 8 as a reactant in a chemical reaction for the introduction of SF₅O⁻ into organic molecules.

10. The use according claim 9 in nucleophilic substitution reaction, wherein the nucleophilic compound is the SF₅O⁻ anion contain in the composition defined in claim 8.
